(19) Europäisches Patentamt
European Patent Office
Office européen des brevets

(11) Veröffentlichungsnummer: **0 352 568**
**A2**

(12) # EUROPÄISCHE PATENTANMELDUNG

(21) Anmeldenummer: **89112931.4**

(22) Anmeldetag: **14.07.89**

(51) Int. Cl.4: **C07C 217/28 , C07C 275/14**

(30) Priorität: **28.07.88 DE 3825637**

(43) Veröffentlichungstag der Anmeldung:
**31.01.90 Patentblatt 90/05**

(84) Benannte Vertragsstaaten:
**AT BE CH DE ES FR GB IT LI NL SE**

(71) Anmelder: **RWE-DEA AKTIENGESELLSCHAFT FÜR MINERALOEL UND CHEMIE**
**Überseering 40**
**D-2000 Hamburg 60(DE)**

(72) Erfinder: **Humbert, Heiko, Dr.**
**Hugo-Klemm-Strasse 23**
**D-2100 Hamburg 90(DE)**
Erfinder: **Hoell, Detlef, Dr.**
**Homberger Strasse 18**
**D-4130 Moers 1(DE)**

(74) Vertreter: **Schupfner, Gerhard D. et al**
**Müller, Schupfner & Gauger Karlstrasse 5**
**Postfach 14 27**
**D-2110 Buchholz/Nordheide(DE)**

(54) **Sekundäre Polyetheramine, ihre Herstellung und Verwendung zur Herstellung von Polyharnstoffen.**

(57) Die Erfindung beschreibt neue sekundäre Polyetherpolyamine, sowie deren Verwendung und Herstellung. Insbesondere werden sekundäre Polyoxypropylendiamine und Polyoxypropylentriamine hinsichtlich ihrer Eigenschaften, Synthese und Anwendungen charakterisiert. Mit dieser Stoffklasse ist der Aufbau einer vollständig polyurethanfreien Polyharnstoff-Verbindung möglich. Dabei führt das Polyoxypropylengerüst zu der gewünschten Flexibilität des Polymers, und die hydrophoben Eigenschaften des Polyoxypropylenkörpers verhindern die Aufnahme von Wasser. Die Amin-Endgruppen ermöglichen wegen ihrer hohen Reaktivität darüber hinaus kurze Reaktionszeiten ohne die Verwendung von Aktivatoren. Dabei läßt die Wahl des Substituenten am Amin-Stickstoff eine Anpassung der Reaktionsgeschwindigkeit dieser Verbindung in weitem Rahmen zu.

EP 0 352 568 A2

## Sekundäre Polyetheramine, ihre Herstellung und Verwendung zur Herstellung von Polyharnstoffen

Der Aufbau hochwertiger Polymerer erfolgt durch die Umsetzung von endständig funktionalisierten Verbindungen, bei den Polyurethanen in der Regel durch die Umsetzung von Polyolen und mehrfachfunktionellen Isocyanaten. Diese variantenreiche Reaktion - Alkohol und Isocyanat - verläuft jedoch vergleichsweise langsam und bedingt dadurch Wartezeiten bei der industriellen Fertigung solcher Materialien. Die Reaktionsgeschwindigkeit kann zwar durch den Zusatz von Aktivatoren (auch Katalysatoren genannt) in einem bestimmten Ausmaß beschleunigt werden, jedoch muß bei der Verwendung von Aktivatoren bzw. Katalysatoren auf mögliche Unverträglichkeiten einzelner Bestandteile des in der Praxis verwendeten Mehrkomponenten-Systems geachtet werden, wie Verfärbungen mit anderen Werkstoffen, die z.B. als Oberflächenmaterial eingesetzt werden. Die in der Regel flüchtigen Aktivatoren verleihen zudem dem Endprodukt eine unerwünschte Geruchskomponente.

Häufig verwendete Einsatzstoffe sind heute Polyoxypropylenpolyole, die den Endprodukten ausgezeichnete mechanische Eigenschaften verleihen. Ein wesentlicher Nachteil dieser Stoffklasse, die geringe Reaktionsgeschwindigkeit bei der Addition an Isocyanate, wird durch die sekundäre Natur der Alkohol-Funktion verursacht.

Die sekundäre Alkohol-Funktion kann mittels der Addition von Ethylenoxid an Polyoxypropylenpolyole in eine primäre Alkohol-Funktion unter Herstellung sogenannter "getippter" Polyole umgewandelt werden. Die gebildeten primären Alkoholgruppen weisen eine wesentlich höhere Reaktivität auf, es ist aber ungünstigerweise der Einsatz einer überproportionalen Menge an Ethylenoxid notwendig, um die sekundären Alkohol-Gruppen möglichst vollständig in primäre zu überführen. Durch den hohen Oxyethylen-Anteil verliert man jedoch die charakteristischen Eigenschaften des reinen Polyoxypropylen-Körpers und die damit verbundenen Vorzüge des verzweigten Alkylgerüsts wie Hydrophobie und geringe Kristallinität.

Wünschenswert sind somit Polymer-Bausteine, bei denen die jeweiligen Vorteile von Polyoxypropylen (geringe Wasseraufnahme) und Polyoxyethylen (hohe Reaktivität) ohne deren Nachteile (geringe Reaktivität bzw. ausgeprägte Hydrophilie) vereint sind.

Ein Teil dieser Forderungen wird durch die Polyesterpolyole erfüllt. Die Reaktivität der primären Alkohol-Funktionen sowie die mechanischen Eigenschaften der polymeren Endprodukte entsprechen den Anforderungen der Praxis. Leider ist jedoch die chemische Resistenz der Estergruppen aufgrund ihrer stark beeinträchtigten Hydrolyse-Stabilität gering. Die reversible Ester-Reaktion führt in Anwesenheit von Wasser zur Rückbildung von Säure- und Alkoholgruppen unter Spaltung des Polymergerüsts und damit zu einer Versprödung des Materials.

Primäre Amine werden seit längerer Zeit als sogenannte Starter und als Kettenverlängerer eingesetzt, siehe Kunststoff-Handbuch, Verlag C. Hanser, München/Wien, 2. Auflage, 1983, Band 7 Polyurethane, Seite 18.

Erst in jüngerer Zeit erfolgt der Einsatz primärer aliphatischer Polyetheramine anstelle der entsprechenden Polyole oder als teilweiser Ersatz der Polyole zur Herstellung von Polyurethanen bzw. Polyharnstoffen.

In der US-PS 4 396 729 sind endständige Amine von Polyetherpolyolen zur Herstellung von "reaction injection molded" (RIM) - Elastomeren offenbart. Dort werden ausschließlich primäre Amine eingesetzt und die Elastomeren-Formteile auf der Accuration (RIM)-Maschine hergestellt. Der Ersatz der Alkohol-Funktion durch aliphatische primäre Amingruppen bewirkt nämlich einen außerordentlich hohen Reaktivitätsanstieg bei der Reaktion mit Isocyanaten. Ihr Einsatz ist daher nur im sogenannten RIM-Prozeß in Maschinen mit spezieller Injektionstechnik möglich.

Aus diesem und anderen Gründen wird der Wunsch nach langsameren Polyharnstoff-Systemen artikuliert, z.B. um das Fließverhalten der Spritzgußmasse zu verbessern, die bei Schußzeiten von 0,8 bis 1,2 Sekunden (R.P. Taylor, F. Sanns, in Polyurethanes World Congress 1987, Proceedings of the FSK/SPI, S. 219) in der Form verteilt werden muß. Weiterhin versprechen langsamere Systeme die Nutzung der bereits vorhandenen Produktionseinrichtungen, allerdings in kürzeren und damit verbesserten Cyclenzeiten als es bei den Polyurethan-RIM-Systemen möglich ist (R.J. G. Dominguez, D.M. Rice, R. A. Grigsby, Jr., in Polyurethanes World Congress 1987, Proceedings of the FSK/SPI, S. 216).

Neben der technologischen Aufgabenstellung, diese schnellen Reaktionen technisch zu beherrschen, bedingen die primären Aminfunktionen einen weiteren Nachteil. Aufgrund der beiden substituierbaren Wasserstoffatome können primäre Amine wie eine difunktionelle Gruppe reagieren. Die primär gebildeten aliphatischen Harnstoff-Derivate können zum Biuret vernetzen.

Neben der kovalenten Vernetzung, wie z.B. durch Biuret-Bildung, wird die Tertiärstruktur (Morphologie) der Polyharnstoffe bzw. Polyurethane auch durch die zeitliche Sequenz der Polymerbildungs-Reaktionen beeinflußt (Kunststoff-Handbuch, Verlag C. Hanser, München/Wien, 2. Auflage, 1983, Band 7 Polyurethane,

Kapitel 2.5.2: Segmentierte Polyurethane, Seite 32 ff). Diese Reaktionsabfolge wird durch die unterschiedliche Reaktivität der verwendeten Verbindungen gegenüber der Isocyanatgruppe festgelegt.

Wie Strukturuntersuchungen an Polyurethanen zeigen, kommt der Ausbildung der Hart- und Weichsegmente im Polymeren zur Erzielung bestimmter Werkstoffeigenschaften eine herausragende Bedeutung zu (J.P. Armistead, G.L. Wilkes, R.B. Turner, Journal of Applied Polymer Science, Vol. 35, 601-629, 1988).

So wurde z.B. für ein Polyharnstoff-RIM-Material eine Veränderung der Abhängigkeit des Biegemoduls vom Hartsegmentanteil gezeigt, bei der eine nichtlineare Abhängigkeit der beiden Größen ab einem bestimmten Hartsegmentanteil in einen linearen Zusammenhang umschlägt (R.A. Grigsby, D.M.Rice, Proceedings of the SPI, 30th Annual Technical/Marketing Conference, 1986, Seite 2). Die Autoren führen dieses Verhalten auf den dort beginnenden dominierenden Einfluß der Hartsegmente auf die Polymer-Morphologie zurück.

Der Erfindung liegt die Aufgabe zugrunde, geeignete Polyätheramine zur Herstellung von Polyharnstoffen bereitzustellen, die einerseits die katalysatorfreie Umsetzung mit den üblichen Isocyanaten gestatten und andererseits die Herstellung von Polyharnstoffen ermöglichen, deren Bildungs- bzw. Verarbeitungsgeschwindigkeit die Umsetzung in konventioneller Verarbeitungstechnik und die gezielte Variation der Polymer-Morphologie erlaubt.

Diese Aufgabe wird erfindungsgemäß durch die Bereitstellung sekundärer Amine der folgenden Formel

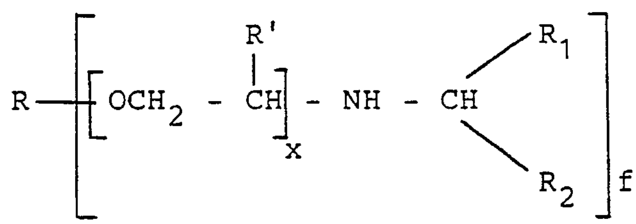 I,

worin

R 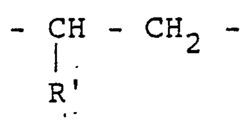

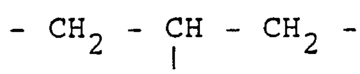

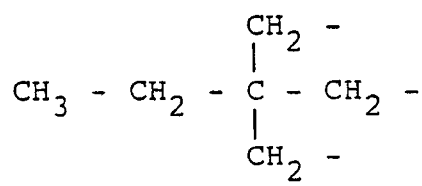 oder

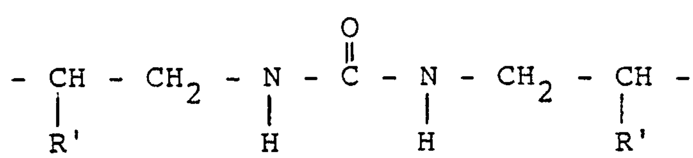 ,

$R_1$ Wasserstoff, geradkettiges, verzweigtes oder cyclisches Alkyl mit 1 bis 6 Kohlenstoffatomen, insbesondere geradkettiges oder verzweigtes Alkyl mit 1 bis 4 Kohlenstoffatomen, besonders bevorzugt Methyl oder 2-Methyl-1-propyl,

$R_2$ verzweigtes Alkyl mit 3 bis 10 Kohlenstoffatomen, Furyl-(2), oder Tetrahydrofuryl-(2), insbesondere verzweigtes Alkyl mit 3 bis 7 Kohlenstoffatomen, besonders bevorzugt 2-Methyl-1-propyl, 2-Propyl oder 2,4-Dimethyl-1-pentyl,

$R_1$ und

$R_2$ zusammen cyclisches Alkyl mit 5 bis 8 Kohlenstoffatomen, ggf. mit Alkyl mit 1 bis 3

Kohlenstoffatomen ein-, zwei- oder dreifach substituiert,
$R'$ Wasserstoff oder Methyl,
x 1-100 und
f 2 oder 3 bedeuten.

Gegenstand der Erfindung ist weiterhin die Herstellung dieser Verbindungen, dadurch gekennzeichnet, daß man Carbonyl-Verbindungen der allgemeinen Formel

$$R_1 - \overset{\overset{\displaystyle O}{\|}}{C} - R_2 \qquad II,$$

worin
$R_1$ Wasserstoff, geradkettiges, verzweigtes oder cyclisches Alkyl mit 1 bis 6 Kohlenstoffatomen, insbesondere geradkettiges oder verzweigtes Alkyl mit 1 bis 4 Kohlenstoffatomen, besonders bevorzugt Methyl oder 2-Methyl oder 2-Methyl-1-propyl,
$R_2$ verzweigtes Alkyl mit 3 bis 10 Kohlenstoffatomen, oder Furyl-(2), oder Tetrahydrofuryl-(2), insbesondere verzweigtes Alkyl mit 3 bis 7 Kohlenstoffatomen, besonders bevorzugt 2-Methyl-1-propyl, 2-Propyl oder 2,4-Dimethyl-1-pentyl
$R_1$ und
$R_2$ zusammen cyclisches Alkyl mit 5 bis 8 Kohlenstoffatomen, ggf. mit Alkyl mit 1 bis 3 Kohlenstoffatomen ein-, zwei- oder dreifach substituiert, bedeuten
mit Polyoxyalkylendi- und triaminen der allgemeinen Formel

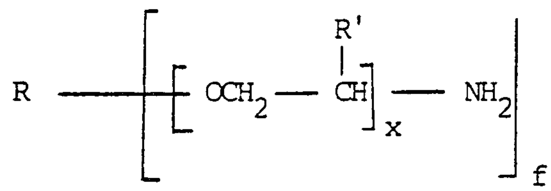
III,

R 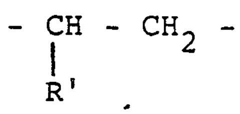 ,

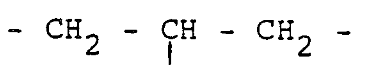 ,

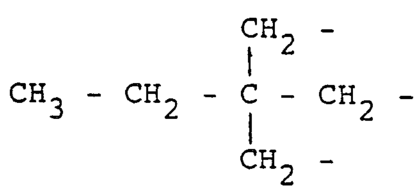 oder

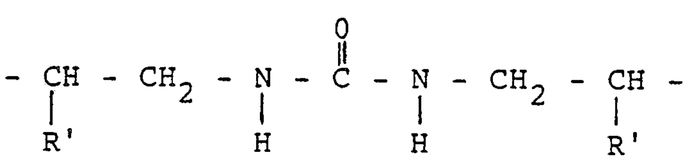 ,

$R'$ Wasserstoff oder Methyl,
x 1-100 und
f 2 oder 3 bedeuten,
pro Moläquivalent der Aminoverbindung mit 1 bis 30 Mol der Carbonylverbindung bei einer Temperatur im Bereich von 55 bis 160 °C bis zur Beendigung der $H_2O$-Bildung umsetzt und das Reaktionsprodukt in Gegenwart von Edelmetallkatalysatoren unter Druck hydriert.

Die Erfindung betrifft weiterhin die Anwendung der Verbindungen zur katalysatorfreien Umsetzung mit Isocyanaten zur Herstellung von Polyharnstoff-Polymerisaten.

Von den erfindungsgemäß hergestellten sekundären Polyetheraminen wird sowohl eine definierte

Funktionalität als auch eine abgemilderte, aber gleichwohl hohe Reaktivität im Vergleich zu primären oder sekundären Alkoholen erreicht. Dabei bürgt das beibehaltene Polyoxypropylengerüst für eine unverändert hohe Elastizität und Hydrolyse-Unempfindlichkeit des Endproduktes.

Die Ausbildung der Phasen (Hart- und Weichsegmente) bei der Bildung der Polymerstruktur hängt wesentlich von der Reaktionsgeschwindigkeit der einzelnen Komponenten mit dem Isocyanat ab. Wie reaktionskinetische Untersuchungen zeigen, besitzen die erfindungsgemäß beanspruchten aliphatischen sekundären Polyetheramine - im Gegensatz zu den um nahezu zwei Zehnerpotenzen schneller reagierenden analogen primären Polyetheraminen - eine Reaktivität, die unerwarteter Weise in einer ähnlichen Größenordnung wie die aromatischer Diamine liegt. Da diese Stoffklasse (z.B. Diethyltoluoldiamin, DETDA) häufig als sogenanner Kettenverlängerer beim RIM-Verfahren eingesetzt wird und maßgeblich die Hartsegmentbildung steuert, eröffnet sich hier unerwartet die Möglichkeit, die Morphologie derartig zusammengesetzter Materialien gezielt zu verändern und ggf. die Dominanz von Weich- bzw. Hartsegmenten zu beeinflussen. Im Vergleich zu den Polymeren aus primären Amin-Monomeren erhält man bei Verwendung der sekundären Polyetheramine aufgrund der nahezu parallel verlaufenden Reaktion von Weich- und Hartsegmentbildnern eine drastisch veränderte Morphologie.

Die hergestellten sekundären Polyetherpolyamine erfüllen in optimaler Weise die heute gestellten Anforderungen an Materialien zum Aufbau von Polymeren. Sie heben sich durch folgende Eigenschaften von den klassischen Polyurethan-Komponenten ab:
hohe Selektivität und hohe Reaktivität
definierte Funktionalität
chemische und mechanische Resistenz
verbesserte thermische Stabilität.

Diese Vorzüge ermöglichen über die dargestellten Verbesserungen hinaus die Verwendung neuer Formulierungen, die sich als maßgeschneidert für sogenannte "moderne" Systeme und die daran gestellten Ansprüche erweisen. Es handelt sich hierbei um Systeme,

- die ohne Aktivator-Zusatz reagieren
- die verbesserte Temperaturbeständigkeit zeigen
- die kurze Cyclenzeiten ermöglichen
- die eine geringe Wasseraufnahme erleiden.

Die gewünschte gezielte Einflußnahme auf die Reaktionsgeschwindigkeit wird bei den erfindungsgemäßen sekundären Polyetheraminen in einfacher Weise durch die Wahl des Substituenten am Amin-Stickstoffatom möglich. Wie die weiter unten beschriebenen kinetischen Messungen an verschieden substituierten Polyoxypropylendiaminen zeigen, ist die Einstellung einer bestimmten Reaktivität dieser Amine ohne weiteres möglich. Die Reaktionsgeschwindigkeit kann in einem Bereich von einer 20 bis 80fachen Verlangsamung der Reaktion mit Isocyanaten beeinflußt werden. Durch Mischen der verschiedenen Komponenten kann eine kontinuierlich veränderbare Einstellung der Reaktivität vorgenommen werden.

In den Anwendungsbeispielen sind die Einsatzbereiche der aus den erfindungsgemäßen sekundären Polyetheraminen hergestellten polyurethanfreien Polyharnstoff-Systeme erläutert. Die konventionellen Verarbeitungsmethoden können aufgrund der strukturellen Variabilität der erfindungsgemäßen sekundären Polyoxypropylenpolyamine ohne weiteres den modernen Erfordernissen der Polyurethan-Technologie angepaßt werden. Daher eignen sie sich auch für alle Anwendungsbereiche der klassischen Polyurethan-Chemie, wie z.B. Hartschaum, Weichschaum, Kaltschaum, Integralschaum, mikrozellularer Schaum, 1K-Schaum, Gießelastomere, RIM-Anwendungen, Dispersionen, Beschichtungen, Sprühbeschichtungen und Kleber.

Besonders geeignet zur Herstellung der erfindungsgemäßen sekundären Polyetheramine sind Carbonyl-Verbindungen, die die folgenden Eigenschaften besitzen:

- einen Siedepunkt bei Normaldruck zwischen 60 und 160 °C
- eine geringe Mischbarkeit mit Wasser
- Bildung eines Minimumazeotrops mit Wasser
- eine Verzweigung in α-oder ß-Stellung zur Carbonyl-Gruppe,

wie z.B.

1. 2-Methylpropanal
2. 2-Methylbutanal
3. 3-Methylbutanal
4. 2,2-Dimethylpropanal
5. 2-Ethylhexanal
6. 3-Methyl-2-butanon
7. 4-Methyl-2-pentanon
8. 2,2-Dimethyl-3-butanon

9. 2,4-Dimethyl-3-pentanon
10. 4,6-Dimethyl-2-heptanon
11. 2,6-Dimethyl-4-heptanon
12. Methylcyclopropylketon
13. Methylcyclopentylketon
14. Methylcyclohexylketon
15. 2-Methylcyclopentanon
16. 2-Methylcyclohexanon
17. 2,5-Dimethylcyclopentanon
18. 2,6-Dimethylcyclohexanon
19. Cyclohexanon
20. Cyclopentanon und davon insbesondere die Carbonylverbindung 6., 7., 10. und 11. oder ein Gemisch von 10. und 11. (= Diisobutylketon, technisch).

Die Carbonylverbindungen der allgemeinen Formel II werden mit primären Polyoxyethylen/polyoxypropylen-diaminen und -triaminen der allgemeinen Formel III umgesetzt.

Diese Polyoxyalkylendi- und triamine haben ein mittleres Molekulargewicht im Bereich von etwa 140 bis 5000, vorzugsweise von etwa 2000 bis 5000 und eine Funktionalität (f) von 2 oder 3. Die Gruppe R repräsentiert den Grundkörper der zur Oxyalkylierung benutzten Verbindungen, wie z.B. Ethylenglykol, Propylenglykol, Glycerin, Trimethylolpropan oder Harnstoff.

Der Rest R′ ist Wasserstoff bei Polyoxyethylen-Verbindungen oder Methyl bei Polyoxypropylen-Verbindungen; der Index x kann Werte von 1 bis 100 annehmen.

Kommerziell erhältliche Verbindungen dieses Typs sind die Polyoxyalkylendiamine und -triamine, die von der Texaco Chemical Company unter dem Handelsnamen Jeffamine verkauft werden, wie z.B.:
Jeffamin® D 4000 (Polyoxypropylendiamin; mittl. Molmasse 4000)
Jeffamin® D 2000 (Polyoxypropylendiamin; mittl. Molmasse 2000)
Jeffamin® T 5000 (Polyoxypropylentriamin; mittl. Molmasse 5000)
Jeffamin® D 400 (Polyoxypropylendiamin; mittl. Molmasse 400)
Jeffamin® D 230 (Polyoxypropylendiamin; mittl. Molmasse 230)
Jeffamin® DU 700 (Harnstoff gestartetes polyoxypropylendiamin; mittl. Molmasse 700)
Jeffamin® T 403 (Polyoxypropylentriamin; mittl. Molmasse 440)
Jeffamin® EDR 148 (Polyoxyethylendiamin; Molmasse 148)
Jeffamin® EDR 192 (Polyoxyethylendiamin; Molmasse 192)

Die Synthese der erfindungsgemäßen sekundären Polyetheramine gelingt in kontrollierter Weise durch einen zweistufigen Prozeß. Im ersten Schritt werden - in einer dem Chemiker an sich bekannten Weise - Ketone oder Aldehyde mit den primären Polyetheraminen zu den Schiffschen Basen (Azomethinen) umgesetzt. Als Schleppmittel zur azeotropen Austragung des gebildeten Wassers dienen entweder die verwendeten Ketone oder Aldehyde selbst oder externe Schleppmittel, die zugesetzt werden. Die Schleppmittel bilden mit Wasser zweiphasige Azeotrope, die aus dem System herausgeführt zerfallen, und nach Abtrennung des Reaktionswassers in den Prozeß zurückgeführt werden. Die Menge des Reaktionswassers erlaubt eine einfache Kontrolle der Vollständigkeit der Umsetzung. Die Siedetemperatur der verwendeten Schleppmittel bzw. der eingesetzten Carbonylverbindungen liegt in einem Bereich zwischen 40 und 160° C. Durch den Zusatz geeigneter Schleppmittel kann eine gewünschte Reaktions- bzw. Siedetemperatur eingestellt werden. Im Interesse der Wirtschaftlichkeit finden solche Ketone bzw. Aldehyde Verwendung, die kommerziell erhältlich sind und über die obengenannten physikalischen Eigenschaften verfügen, wie z. B. 4-Methyl-2-pentanon (MIBK), 3-Methyl-2-butanon (MIK), die technisch hergestellte Mischung aus 4,6-Dimethyl-2-heptanon und 2,6-Dimethyl-4-heptanon (DIBK), Cyclohexanon, Furfurol und 2-Methyl-propanal (iso-Butyraldehyd).

Pro Moläquivalent der Aminogruppe werden 1 bis 30 Mol der Carbonylverbindung bei Temperaturen von 55 bis 160° C, mindestens der Siedetemperatur der eingesetzten Carbonylverbindungen und höchstens der Zersetzungstemperatur der Verfahrenskomponenten, 2 bis 40 h bis zur Beendigung der Waserbildung umgesetzt.

Die Hydrierung der gebildeten Schiffschen Basen wird -in einer dem Fachmann an sich bekannten Weise - mit Edelmetall-Katalysatoren wie Pd, Pt oder Re in kontinuierlicher oder diskontinuierlicher Fahrweise durchgeführt. Palladium-Katalysatoren eignen sich in besonderer Weise, da eine hydrogenolytische Spaltung der Etherbindungen verhindert wird und damit die Hydrierung der CN-Doppelbindung mit hoher Selektivität verläuft. Als Träger kann sowohl mineralisches Material wie Aluminiumoxid als auch Kohle in Form von Granulat, Pellets oder pulverisiert verwendet werden. Die Konzentration des Katalysators,

bezogen auf das eingesetzte Hydriergut beträgt 0,1 bis 5 Gew.%.

Bei einer diskontinuierlichen Fahrweise verwendet man insbesondere einen Rührautoklaven, der mit etwa 0,5 Gew.% Katalysator bezogen auf eingesetztes Hydriergut und mit der Schiffschen Base in Substanz oder in einem Lösungsmittel, vorzugsweise in dem zur Umsetzung verwendeten Keton/Aldehyd und/oder dem entsprechenden sekundären Alkohol, der hydrierten Ausgangskomponente, befüllt wird. Bei einem Druck von 60 bis 180 bar, bevorzugt bei 100 bis 140 bar, und einer Temperatur zwischen 100 und 200 °C, bevorzugt bei 140 bis 160 °C, wird die Hydrierung ausgeführt.

Bei der kontinuierlichen Hydrierung etwa im Rieselreaktor werden ebenfalls Edelmetall-Katalysatoren der oben genannten Art eingesetzt. Ein typischer Katalysator hat den Gehalt von 5 % Gew.% Pd bezogen auf Aluminiumoxid. Geeignete Reaktionsbedingungen sind Temperaturen von 100 bis 220 °C, bevorzugt 150 bis 175 °C, ein Druck von 60 bis 120 bar, bevorzugt 100 bar und eine Raumzeitgeschwindigkeit (LHSV = liquid hourly space velocity) von 1 l Flüssigkeitsvolumen/h . l Reaktorvolumen.

Die folgenden Beispiele erläutern die Herstellung und Verwendung der erfindungsgemäßen Verbindungen. Die angegebenen Molmassen sind jeweils Werte im statistischen Mittel.

## Beispiel 1

N,N′-Bis(4-methyl-2-pentyl)-polyoxypropylendiamin -Molmasse 390

Einwaagen : 87,6 kg Polyoxypropylendiamin mit einer Molmasse von 230 -Jeffamin® D 230 152,5 kg 4-Methyl-2-pentanon

Gesamtmenge : 240,1 kg

Die Einsatzstoffe wurden gemeinsam in einen mit Rührwerk versehenen Reaktor chargiert und zum Sieden erhitzt. Bei einer Sumpftemperatur von ca. 115 °C begann die Wasserabscheidung. Das übergehende 4-Methyl-2-pentanon wurde in den Reaktor zurückgeführt. Das Reaktionsende war anhand der gebildeten Wassermenge erkennbar (ca. 10,5 l); Reaktionsdauer 29 h.

Sodann wurde bei Normaldruck das überschüssige 4-Methyl-2-pentanon abdestilliert. Die Sumpftemperatur stieg dabei von 139 °C bis auf 161 °C. Nach 6 h verblieben 165 kg des Azomethins, das direkt zur Hydrierung eingesetzt wurde.

In einer kontinuierlich betriebenen Hydrierapparatur mit einem Volumen von 4 l und einer Katalysatorzone von 9 m, befüllt mit 5 % Pd auf $Al_2O_3$, wurde das Zwischenprodukt innerhalb von 50 h bei einer Temperatur von 151-153 °C und einem Druck von 100 bar hydriert. Das so erhaltene Rohprodukt wurde bei 112 °C und 24 mbar 10 h lang unter $N_2$-Durchleitung von flüssigen Bestandteilen befreit.

Es verblieben 141 kg N,N′- Bis (4-methyl-2-pentyl)-polyoxypropylendiamin (Molmasse 390).

Physikalische Daten:

Gesamt-Amingehalt : 5,1 mval/g
Brechungsindex : $n_D$20 1,4436
Wassergehalt : 0,2 %
Viskosität : 13 mPa.s

## Beispiel 2

N,N′- Bis (4-methyl-2-pentyl)-polyoxypropylendiamin -Molmasse 560

Einwaagen : 42,6 kg Polyoxypropylendiamin mit einer Molmasse von 400 -Jeffamin® D 400 53,4 kg 4-Methyl-2-pentanon

Gesamtmenge : 96,0 kg

Die Einsatzstoffe wurden gemeinsam befüllt und zum Sieden erhitzt. Bei einer Sumpftemperatur von ca. 110 °C begann die Wasserabscheidung. Das abdestillierende 4-Methyl-2-pentanon wurde in den Reaktor zurückgeführt. Das Reaktionsende war anhand der gebildeten Wassermenge erkennbar (ca. 3,4 l): Reaktionsdauer 22 h.

Sodann wurde bei Normaldruck das überschüssige 4-Methyl-2-pentanon abdestilliert. Die Sumpftemperatur stieg dabei von 146 °C bis auf 163 °C. Nach 3 h verblieben 65 kg des Azomethins, das direkt zur Hydrierung eingesetzt wurde.

In einer kontinuierlich betriebenen Hydrierapparatur (wie in Beispiel 1 beschrieben) wurde das Zwischenprodukt innerhalb von 100 h bei einer Temperatur von 150 °C und einem Druck von 100 bar hydriert.

Das so erhaltene Rohprodukt wurde bei 160 °C und 25 mbar 7 h lang unter $N_2$-Durchleitung von flüchtigen Bestandteilen befreit. Es verblieben 56 kg des Produkts N, N′-Bis (4-methyl-2-pentyl)-polyoxypropylendiamin (Molmasse 560).

Physikalische Daten:

Gesamt-Amingehalt : 3,2 mval/g
Brechungsindex : $n_D20$ 1,4464
Wassergehalt : 0,1 %
Viskosität : 33 mPa.s

Beispiel 3

N,N′-Bis(4-methyl-2-pentyl)-polyoxypropylendiamin-Molmasse 2150

Einwaagen : 138,6 kg Polyoxypropylendiamin mit einer Molmasse von 2000 -Jeffamin® D 2000 101,4 kg 4-Methyl-2-pentanon
Gesamtmenge : 240,0 kg

Die Einsatzstoffe wurden gemeinsam befüllt und zum Sieden erhitzt. Bei einer Sumpftemperatur von ca. 96 °C begann die Wasserabscheidung. Das übergehende 4-Methyl-2-pentanon wurde in den Reaktor zurückgeführt. Das Reaktionsende war anhand der gebildeten Wassermenge erkennbar (ca. 2 l); Reaktionsdauer 16 h.

Sodann wurde bei Normaldruck das überschüssige 4-Methyl-2-pentanon abdestilliert. Die Sumpftemperatur stieg dabei von 127 °C bis auf 160 °C. Nach 5 h verblieben 164 kg des Azomethins im Rührwerk. Das Zwischenprodukt wurde direkt zur Hydrierung eingesetzt.

In einer kontinuierlich betriebenen Hydrierapparatur (wie in Beispiel 1 beschrieben) wurde das Zwischenprodukt innerhalb von 47 h bei einer Temperatur von 167 bis 168 °C und einem Druck von 100 bar hydriert. Das so erhaltene Rohprodukt wurde bei 112 °C und 23 mbar 6 h lang unter $N_2$-Durchleitung von flüchtigen Bestandteilen befreit. Es verblieben 140 kg des Produkts N,N′-Bis(4-methyl-2-pentyl)-polyoxypropylendiamin (Molmasse 2150).

Physikalische Daten:

Gesamt-Amingehalt : 0,93 mval/g
Brechungsindex : $n_D^{20}$ 1,4497
Wassergehalt : 0,05 %
Viskosität : 350 mPa.s

Beispiel 4

N,N′,N″-Tris(4-methyl-2-pentyl) -polyoxypropylentriamin-Molmasse 700

Einwaagen : 93,16 kg Trimethylolpropan-polyoxypropylenaddukt-triamin mit einer Molmasse von 440 -Jeffamin® T 403 146,84 kg 4-Methyl-2-pentanon
Gesamtmenge : 240,00 kg

Die Einsatzstoffe wurden gemeinsam befüllt und zum Sieden erhitzt. Bei einer Sumpftemperatur von ca. 115 °C begann die Wasserabscheidung. Das übergehende 4-Methyl-2-pentanon wurde in den Reaktor zurückgeführt. Das Reaktionsende war anhand der gebildeten Wassermenge erkennbar (ca 9 Liter); Reaktionsdauer 26 h.

Sodann wurde bei Normaldruck das überschüssige 4-Methyl-2-pentanon abdestilliert. Die Sumpftemperatur stieg dabei von 127° C bis auf 160° C. Nach 8 h verblieben 154 kg des Azomethins. Das Zwischenprodukt wurde direkt zur Hydrierung eingesetzt.

In einer kontinuierlich betriebenen Hydrierapparatur (wie in Beispiel 1 beschrieben) wurde das Zwischenprodukt innerhalb von 48 h bei einer Temperatur von 169 bis 171° C und einem Druck von 100 bar hydriert. Das so erhaltene Rohprodukt wurde bei 108° C und 25 mbar 7 h lang unter $N_2$-Durchleitung von flüchtigen Bestandteilen befreit. Es verblieben 137 kg des Produkts N,N,N″-Tris(4-methyl-2-pentyl)-polyoxypropylentriamin (Molmasse 700).

Physikalische Daten:

Gesamt-Amingehalt : 4,3 mval/g
Brechungsindex : $n_D^{20}$ 1,4526
Wassergehalt : 0,02 %
Viskosität : 84 mPa.s

Beispiel 5

N,N′,N″-Tris(4-methyl-2-pentyl)-polyoxypropylentriamin -Molmasse 5250

Einwaagen : 143,0 kg Trimethylolpropan-polyoxypropylenaddukt-triamin mit einer Molmasse von 5000 -Jeffamin® T 5000 97,0 kg 4-Methyl-2-pentanon
Gesamtmenge : 240,0 kg

Die Einsatzstoffe wurden gemeinsam befüllt und zum Sieden erhitzt. Bei einer Sumpftemperatur von ca. 119° C begann die Wasserabscheidung. Das übergehende 4-Methyl-2-pentanon wurde in den Reaktor zurückgeführt. Das Reaktionsende war anhand der gebildeten Wassermenge erkennbar (ca. 1,2 Liter); Reaktionsdauer 6 h.

Sodann wurde bei Normaldruck das überschüssige 4-Methyl-2-pentanon abdestilliert. Die Sumpftemperatur stieg dabei von 124° C bis auf 160° C. Nach 6 h verblieben 161 kg des Azomethins. Das Zwischenprodukt wurde nach Zusatz von 15 l 4-Methyl-2-pentanol zur Hydrierung eingesetzt.

In einer kontinuierlich betriebenen Hydrierapparatur (wie im Beispiel 1 beschrieben) wurde das Zwischenprodukt innerhalb von 197 h bei einer Temperatur von 169 bis 170° C und einem Druck von 100 bar hydriert. Das so erhaltene Rohprodukt wurde bei 110° C und 25 mbar 4 h lang unter $N_2$-Durchleitung von flüchtigen Bestandteilen befreit. Es verblieben 136 kg an N,N′, N″-Tris(4-methyl-2-pentvl)-polyoxypropylentriamin (Molmasse 5250).

Physikalische Daten:

Gesamt-Amingehalt : 0,5 mval/g
Brechungsindex : $n_D^{20}$ 1,4519
Wassergehalt : 0,03 %
Viskosität : 1150 mPa.s

Die folgenden sekundären Polyetheramine wurden entsprechend hergestellt, wobei jedoch die Hydrierung chargenweise erfolgte.

Beispiel 6

N,N′-Bis(cyclohexyl)-polyoxypropylendiamin -Molmasse 560

Hergestellt durch Umsetzung von Jeffamin® D 400 mit Cyclohexanon.
Gesamt-Amingehalt : 3,15 mval/g
Brechungsindex : $n_D^{20}$ 1,4660
Wassergehalt : 0,04 %
Viskosität : 89 mPa.s

Beispiel 7

N,N′-Bis(cyclohexyl)-polyoxypropylendiamin -Molmasse 2150

Hergestellt durch Umsetzung von Jeffamin® D 2000 mit Cyclohexanon.
Gesamt-Amingehalt : 0,90 mval/g
Brechungsindex : $n_D^{20}$ 1,4556
Wassergehalt : 0,01 %
Viskosität : 450 mPa.s

Beispiel 8

N,N′-Bis(isononyl)-polyoxypropylendiamin -Molmasse 2250

Hergestellt durch Umsetzung von Jeffamin® D 2000 mit Diisobutylketon (technisch).
Gesamt-Amingehalt : 0,90 mval/g
Brechungsindex : $n_D^{20}$1,4509
Wassergehalt : 0,01 %
Viskosität : 392 mPa.s

Beispiel 9

N,N′-Bis(3-methyl-2-butyl)-polyoxypropylendiamin -Molmasse 2120

Hergestellt durch Umsetzung von Jeffamin® D 2000 mit 3-Methyl-2-butanon.
Gesamt-Amingehalt : 0,85 mval/g
Brechungsindex : $n_D^{20}$ 1,4505
Wassergehalt : 0,1 %
Viskosität : 336 mPa.s

Beispiel 10

N,N′-Bis(2-methyl-1-propyl)-polyoxypropylendiamin Molmasse 2100

Hergestellt durch Umsetzung von Jeffamin® D 2000 mit iso-Butyraldehyd.
Gesamt-Amingehalt : 0,86 mval/g
Brechungsindex : $n_D^{20}$ 1,4511
Wassergehalt : 0,08 %
Viskosität : 391 mPa.s

Beispiel 11

N,N′-Bis(4-methyl-2-pentyl)-polyoxypropylendiamin Molmasse 390

Hergestellt durch Umsetzung von Jeffamin® D 230 mit 4-Methyl-2-pentanon.
Gesamt-Amingehalt : 5,0 mval/g
Brechungsindex : $n_D^{20}$ 1,4443
Wassergehalt : 0,13 %
Viskosität : 12 mPa.s

Beispiel 12

N,N′-Bis(4-methyl-2-pentyl)-polyoxypropylendiamin -Molmasse 560

Hergestellt durch Umsetzung von Jeffamin® D 400 mit 4-Methyl-2-pentanon.
Gesamt-Amingehalt : 3,26 mval/g
Brechungsindex : $n_D^{20}$ 1,4463
Wassergehalt : 0,08 %
Viskosität : 32 mPa.s

Beispiel 13

N,N′-Bis(4-methyl-2-pentyl)-polyoxypropylendiamin -Molmasse 2150

Hergestellt durch Umsetzung von Jeffamin® D 2000 mit 4-Methyl-2-pentanon.
Gesamt-Amingehalt : 0,86 mval/g
Brechungsindex : $n_D^{20}$ 1,4505
Wassergehalt : 0,14 %
Viskosität : 300 mPa.s

Beispiel 14

N,N′-Bis(4-methyl-2-pentyl)-polyoxypropylendiamin -Molmasse 4140

Hergestellt durch Umsetzung von Jeffamin® D 4000 mit 4-Methyl-2-pentanon.
Gesamt-Amingehalt : 0,43 mval/g
Brechungsindex : $n_D^{20}$ 1,4508
Wassergehalt : 0,01 %
Viskosität : 1173 mPa.s

Beispiel 15

N,N′, N″-Tris(4-methyl-2-pentyl)-polyoxypropylentriamin -Molmasse 700

Hergestellt durch Umsetzung von Jeffamin® T 403 mit 4-Methyl-2-pentanon.
Gesamt-Amingehalt : 4,3 mval/g
Brechungsindex : $n_D^{20}$ 1,4532
Wassergehalt : 0,1 %
Viskosität : 88 mPa.s

Beispiel 16

N,N′, N″-Tris(4-methyl-2-pentyl)-polyoxypropylentriamin-Molmasse 5250

Hergestellt durch Umsetzung von Jeffamine® T 5000 mit 4-Methyl-2-pentanon.
Gesamt-Amingehalt : 0,45 mval/g
Brechungsindex : $n_D^{20}$ 1,4518
Wassergehalt : 0,15 %
Viskosität : 1106 mPa.s

Beispiel 17

Kinetische Messungen

Vergleich von N,N′-Bis(4-methyl-2-pentyl)-polyoxypropylen diamin (Molmasse 2150), N,N′-Bis(cyclohexyl)-polyoxypropylendiamin (Molmasse 2150) und N,N′-Bis(isononyl)-polyoxypropylendiamin (Molmasse 2250) mit Jeffamin® D 2000 bei der Reaktion mit Phenylisocyanat.

Mittels der IR-Spektroskopie kann die Reaktionsgeschwindigkeit der Umsetzung von Isocyanaten mit alkylierten Polyetheraminen sowie mit den entsprechenden primären Polyetheraminen bestimmt werden. Dazu wurde eine Phenylisocyanat-Lösung mit bekannter Konzentration mit einer eingestellten Lösung des entsprechenden Polyetheramins vermischt und die Abnahme der Isocyanat-Absorption IR-spektroskopisch verfolgt.

Wegen der außerordentlich schnell ablaufenden Reaktion mußte eine ausreichende zeitliche Auflösung der Reaktion durch die Wahl einer entsprechenden Verdünnung erreicht werden. Die eingesetzten Lösungen hatten folgende Konzentrationen:
Phenylisocyanat-Lösung: 0,006 mol/l
Polyetherdiamin-Lösung: 0,002 mol/l.

Das durch die hohe Verdünnung bedingte Empfindlichkeitsproblem bei der Aufnahme des IR-Spektrums wurde durch die Verwendung eines geeigneten Lösungsmittels ($CCl_4$) minimiert. In der Nähe der Isocyanat-Absorption bei einer Wellenzahl von $v = 2260\ cm^{-1}$ zeigt Tetrachlorkohlenstoff keine Absorption.

Die Reaktionsmischung wurde durch Vermischen von 1,5 Teilen Phenylisocyanat-Lösung und 1 Teil Polyetherdiamin-Lö sung hergestellt. Daraus ergeben sich folgende Startwerte bei der Umsetzung:
Phenylisocyanat-Konzentration: 0,0036 mol/l
Polyetherdiamin-Konzentration: 0,0008 mol/l.

Bei der Auswertung der gewonnenen Ergebnisse wurde vorausgesetzt, daß die Aminogruppen des Polyetherdiamins wie zwei unabhängige, reaktive Gruppen in einer bimolekularen Reaktion abreagieren.

Im einzelnen wurden folgende Verbindungen untersucht und miteinander verglichen:
N,N′-Bis(4-methyl-2-pentyl)-polyoxypropylendiamin (Molmasse 2150)
N,N′-Bis(cyclohexyl)-polyoxypropylendiamin (Molmasse 2150)
N,N′-Bis(isononyl)-polyoxypropylendiamin (Molmasse 2250)
Jeffamin® D 2000 primäres Polyetherdiamin (Molmasse 2000).

Bei der Auswertung wurde der Geschwindigkeitskoeffizient aus der Steigung der logarithmisch aufgetragenen Daten gewonnen.

Folgende Geschwindigkeitskoeffizienten wurden nach einer Regressionsanalyse der umgerechneten Meßwerte erhalten:
N,N′-Bis(4-methyl-2-pentyl)-polyoxypropylendiamin (Molmasse 2150):
$k = 2{,}4 \pm 0{,}3\ l.mol^{-1}s^{-1}$
N,N′-Bis(cyclohexyl)-polyoxypropylendiamin (Molmasse 2150):
$k = 5{,}8 \pm 1{,}6\ l.mol^{-1}s^{-1}$
N,N′-Bis(isononyl)-polyoxypropylendiamin (Molmasse 2250):
$k = 1{,}8 \pm 0{,}3\ l.mol^{-1}s^{-1}$

Eine Messung der Isocyanat-Reaktion des primären Poly etherdiamins war aufgrund der zu kurzen Reaktionsdauer nicht möglich. Die Geschwindigkeitskonstante des primären Diamins (Jeffamin® D 2000) kann jedoch zu $k \geqq 30\ l\ .\ mol^{-1}s^{-1}$ abgeschätzt werden.

Gegenüber der unsubstituierten Verbindung erreicht man bei den erfindungsgemäßen sekundären Polyetheraminen eine mindestens um den Faktor 5 bis 10 verlängerte Reaktionszeit. Dabei ist durch die Wahl des Alkylsubstituenten eine gezielte Variation der Reaktionszeit möglich.

## Ansprüche

1. Sekundäre Polyetheramine der allgemeinen Formel

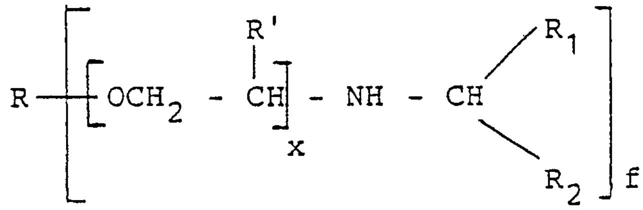 I,

worin

R 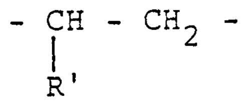

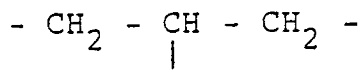

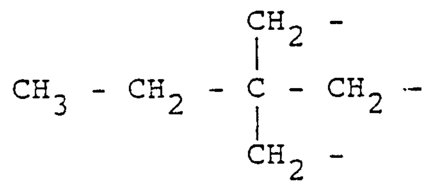 oder

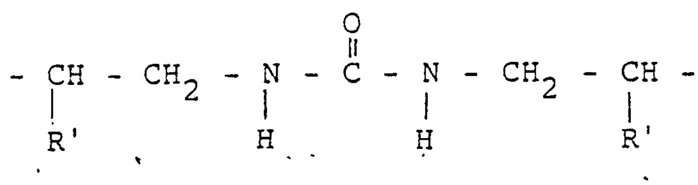 ,

$R_1$ Wasserstoff, geradkettiges, verzweigtes oder cyclisches Alkyl mit 1 bis 6 Kohlenstoffatomen,
$R_2$ verzweigtes Alkyl mit 3 bis 10 Kohlenstoffatomen, Furyl-(2) oder Tetrahydrofuryl-(2),
$R_1$ und
$R_2$ zusammen cyclisches Alkyl mit 5 bis 8 Kohlenstoffatomen, ggf. mit Alkyl mit 1 bis 3 Kohlenstoffatomen ein-, zwei- oder dreifach substituiert,
$R'$ Wasserstoff oder Methyl,
x 1 -100 und
f 2 oder 3 bedeuten.

2. Verfahren zur Herstellung sekundärer Polyetheramine des Anspruch 1,

**dadurch gekennzeichnet,**

daß man Carbonyl-Verbindungen der allgemeinen Formel

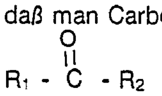 II,

worin
$R_1$ Wasserstoff, geradkettiges, verzweigtes oder cyclisches Alkyl mit 1 bis 6 Kohlenstoffatomen,
$R_2$ verzweigtes Alkyl mit 3 bis 10 Kohlenstoffatomen, Furyl-(2), oder Tetrahydrofuryl-(2),
$R_1$ und
$R_2$ zusammen cyclisches Alkyl mit 5 bis 8 Kohlenstoffatomen, ggf. mit Alkyl mit 1 bis 3 Kohlenstoffatomen ein-, zwei- oder dreifach substituiert, bedeuten,
mit Polyoxyalkylendi- und triaminen der allgemeinen Formel

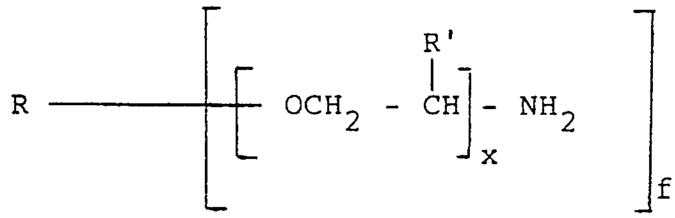 III,

worin

R 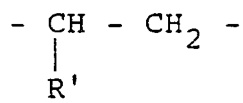

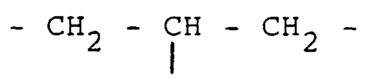 ,

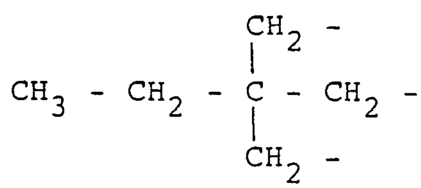 oder

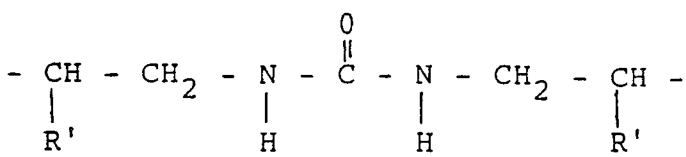 ,

R′ Wasserstoff oder Methyl,

x 1-100 und

f 2 oder 3 bedeuten,

pro Moläquivalent der Aminoverbindung mit 1 bis 30 Mol der Carbonylverbindung bei einer Temperatur im Bereich von 55 bis 160 °C bis zur Beendigung der $H_2O$-Bildung umsetzt und das Reaktionsprodukt in Gegenwart von Edelmetallkatalysatoren unter Druck hydriert.

3. Verwendung der Verbindungen des Anspruchs 1 zur katalysatorfreien Umsetzung mit Isocyanaten zur Herstellung von Polyharnstoffen.